# EUROPEAN PATENT APPLICATION

(11) **EP 3 312 288 A1**
(43) Date of publication of application: **25.04.2018**
(21) Application number: 16194477.2
(22) Date of filing: 18.10.2016
(51) Int. Cl.: C12Q 1/04, C12Q 1/18, C12N 1/20

(54) **METHOD, MEDIUM AND KIT FOR DETECTING MULTIPLE AMINOGLYCOSIDE RESISTANCE INCLUDING 16S RRNA METHYLASE MEDIATED RESISTANCE IN GRAM-NEGATIVE BACTERIA**

(71) Applicant: Universite De Fribourg, 1700 Fribourg (CH)
(72) Inventor: NORDMANN, Patrice, 1700 Fribourg (CH); POIREL, Laurent, 1700 Fribourg (CH); JAYOL, Aurélie, 33700 Mérignac (FR)
(74) Representative: Schneiter, Sorin

(57) **Abstract**

The present invention concerns a method, a test kit and a culture medium for testing for Gram negative bacteria exhibiting multiple resistance to aminoglycoside antibiotics, wherein the method, test kit and culture medium detect resistance conferred by the presence of 16S rRNA methylase while discriminating against aminoglycoside resistance due to other narrowspectrum mechanisms.

## Description

### Technical Field

The present invention relates to a method, a medium and/or a test for detecting Gram negative bacteria exhibiting multiple resistance to aminoglycoside antibiotics, wherein said resistance is conferred specifically by the presence of 16S rRNA methylases.

### Background Art and Problems Solved by the Invention

Multidrug resistance (MDR) is now emerging at an alarming rate worldwide. The rise of antibiotic-resistant bacterial strains represents a serious threat to public health and the economy. The severity of this menace is amplified by the fact that research for new antibiotic agents is currently stalled. It may be possible that no new agent active against multidrug resistant bacteria will be put on the market in a close future. The 20^{th} century was the "century of antibiotics", marked by the discovery and the continuous development of new more and more active antibiotics, but no new antibiotic family has been available for clinicians since 1987. In a world with few effective antibiotics, modem medical advances such as intensive care, transplant and chemotherapy (cancer treatment) may no longer be possible due to the threat of untreatable infections.

Most of the public bodies such as the World Health Organization (2014), the UK Government (2014), the Center for Disease Control and Prevention in the USA (2013) and the Davos Economic Forum in Switzerland (since 2013) have pull the alarm signal to try to control this complex health issue. It is estimated that 25,000 patients die each year in Europe due to multidrug resistance.

As underlined by the latest and important report from the White House in the US (September 2014, National Strategy for Combating Antibiotic Resistant Bacteria) a multiple facet approach is needed. Basically, this strategy is intended to reach four synergistic goals (i) antibiotic stewardship from agriculture to human medicine, (ii) surveying emerged and emerging resistance determinants (iii) accelerate basic and applied research and the development for new antibiotics, other therapeutics and vaccines, (iv) and advance development and use of rapid and innovative diagnostic tests for identification and characterization of resistant bacteria.

Infections are mostly due to either Gram positives such as *Staphyloccus aureus* and Gram negatives such as *Escherichia coli.* Most of the important resistance issues are now focused in Gram negatives for which, in addition, there is a lack of novel antimicrobial agents. They are the main causes of infections in humans. They are the source of both community-acquired and hospital-acquired infections (urinary tract infections, septicemia, intra-abdominal infections...). By far, clinically-significant Gram negatives are the *Enterobacteriaceae* (*E. coli, Klebsiella pneumoniae, Salmonella...).*

Extended-spectrum ß-lactamases that hydrolyze extended-spectrum cephalosporins, and carbapenemases that hydrolyze in addition carbapenems are disseminating worldwide in *Enterobacteriaceae,* and therapeutic options are becoming limited (1). In those multidrug resistant isolates, aminoglycosides (AG) may still be considered as valuable treatment options (2), but their efficacy has been reduced by the surge and the dissemination of resistance.

The most prevalent mechanism of resistance to AG in *Enterobacteriaceae* are enzymes, which modify the chemical structure of aminoglycosides and can be nucleotidyltransferases, phosphotransferases, or acetyltransferases (3). Those aminoglycoside-modifying enzymes are often present in various combinations in a given strain and are typically found on plasmids.

However, plasmid-mediated 16S rRNA methylases conferring a high level of resistance to multiple AG is also reported (4), and they are identified at a high frequency in particular among producers of the metallo-carbapenemases NDM (5). Enzymes encoded by these genes methylate the aminoglycoside-tRNA recognation site (A-site) of the 16S rRNA, which are the intracellular AG target of the aminoglycosides. The 16S rRNA methylases described in *Enterobacteriaceae* are ArmA, RmtB to RmtF, and NpmA, with ArmA being the most frequently identified (4). Those methylases exhibit significant amino acid heterogeneity, although sharing similar amino acid motives in their active sites.

In an area of paucity of novel molecules, identifying rapidly multiple resistance to AG by a simple test may be useful for implementing antibiotic stewardship and containment of those MDR bacteria.

It is an objective of the present invention to provide a method, test, kit and a selection medium suitable to rapidly detect Gram negatives exhibiting multiple resistance to aminoglycosides, and in particular AG resistant *Enterobacteriaceae* in which resistance is conferred by the presence of 16S rRNA methylases.

It is an objective of the present invention to specifically detect strains exhibiting 16S rRNA methylase-mediated resistance, and to disregard and/or discriminate against other mechanisms conferring resistance to AGs, such as the presence of aminoglycoside-modifying enzymes and/or reduced uptake or decreased cell permeability.

### Summary of the Invention

The present inventors provide a method, a test and a selective medium for testing for Gram negatives exhibiting multiple resistance to aminoglycoside antibiotics. In particular, the invention relates to the detection of the presence of rRNA methylase-mediated resistance, while discriminating against aminoglycoside resistance due to other mechanisms, such as aminoglycoside-modifying enzymes and/or reduced uptake or decreased cell permeability.

Remarkably, by providing a test, medium and/or solution comprising at least two different aminoglycoside antibiotics, the method of the invention allows detecting specifically the presence of rRNA methylase-mediated resistance, whereas strains exhibiting other resistance mechanisms are not selected or detected in accordance with the invention.

In an aspect, the method of the invention comprises: providing a test, medium and/or solution comprising at least two different aminoglycoside antibiotics and nutrients for supporting the growth of Gram negative bacteria, in particular *Enterobacteriaceae;* inoculating said medium and/or solution with a sample comprising bacteria; after incubation, screening for bacterial growth and/or metabolic activity in said medium and/or solution; finding a positive test outcome indicating said multiple aminoglycoside resistance due to rRNA methylase if bacterial growth and/or metabolic activity is detected.

In an aspect, the test, kit or medium of the present invention comprises: at least two different aminoglycoside antibiotics and nutrients for supporting the growth of Gram negative bacteria, in particular *Enterobacteriaceae.*

In an aspect, the test, kit or medium of the present invention comprises: at least two different aminoglycoside antibiotics and a carbohydrate source that can be metabolized by the bacteria to be selected.

Further aspects and preferred embodiments of the invention are defined herein below and in the appended claims. Further features and advantages of the invention will become apparent to the skilled person from the description of the preferred embodiments given below.

### Brief Description of the Drawings

**Figure 1** shows representative results of a rapid diagnostic test in accordance with an embodiment of the present invention.
**Figures 2** **A and B** show a selective chromogenic medium according to an embodiment of the invention, on which *Enterobacteriaceae* isolates with 16S rRNA methylases can be differentiated according to their color.
**Figure 3** shows a Drigaliski-containing medium, in accordance with an embodiment of the invention, yielding similar results as with the EMB medium.

Hereinafter, preferred embodiments of the device of the invention are described, in order to illustrate the invention, without any intention to limit the scope of the present invention.

### Detailed Description of the Preferred Embodiments

The present invention relates to screening, testing, detecting and/or selecting bacteria exhibiting multiple resistance to aminoglycoside antibiotics. Preferably, the invention relates to testing for Gram negative bacteria exhibiting resistance to aminoglycoside antibiotics. In an embodiment, the invention relates to testing for Gram negative, aerobic and/or facultative anaerobe bacteria. Preferably, the method detects resistance conferred by the presence of methylases, but preferably does not detect bacteria exhibiting aminoglycoside resistance due to one or more other mechanisms.

In a preferred embodiment, the method, test, medium or solution of the invention allows detecting resistance due to methylase activity. Said methylase activity is preferably rRNA methylase activity, most preferably 16S rRNA methylase activity.

Preferably, the method, test, solution and/or medium of the invention discriminates rRNA methylase-mediated resistance against aminoglycoside resistance due to other mechanisms, such as aminoglycoside-modifying enzymes and/or reduced uptake or decreased cell permeability.

In an embodiment, the method, test, solution and medium of the invention provides a negative test outcome if said bacteria exhibit aminoglycoside resistance exclusively due to aminoglycoside modifying activity, in particular selected from nucleotidyltransferase, phosphotransferase and acetyltransferase activity, and if said bacteria exhibit no aminoglycoside resistance.

The negative test also expected if the aminoglycoside modifying activity in a resistant strain is such that different aminoglycoside antibiotics are modified, as long as 16S rRNA methylase activity is absent.

Preferably, a negative test outcome is obtained if the bacteria that are tested contain only one or more aminoglycoside modifying enzymes, which, altogether are not capable to provide resistance to all of said at least two aminoglycoside antibiotics. A negative test outcome is preferably also obtained if the bacteria that are tested do not contain any resistance to aminoglycoside antibiotics.

Preferably, a negative test outcome is obtained if the bacteria that are tested are resistant to an aminoglycoside antibiotic, wherein said resistance is not due to the presence of a 16S rRNA methylase.

Preferably, a negative test outcome is obtained if the bacteria that are tested are resistant to an aminoglycoside antibiotic, wherein said resistance is due to only one, only two or more aminoglycoside modifying enzymes active in said bacteria, and/or if said resistance is due to reduced uptake or decreased cell permeability with respect to at least one and preferably only one aminoglycoside antibiotic.

In some embodiments, a positive test outcome may be obtained only under the condition that the bacteria that is tested is a gram-negative fermenter, that is a bacterium that is capable of metabolizing glucose, lactose, sucrose, or any other carbohydrate source that is preferably present in the test, medium and/or solution of the invention. In an embodiment, the test, medium and/or solution comprises a source of carbohydrates and is suitable to detect the presence of Gram negative fermenters, preferably *Enterobacteriaceae* exhibiting the resistance to aminoglycosides due to rRNA methylase as specified elsewhere. In an embodiment, the test, medium and/or solution does not allow detecting the presence of non-fermenting Gram negative bacteria, even if they have the RNA methylase-mediated resistance as specified elsewhere.

In another embodiment, in particular where the test outcome is determined by detecting the presence of colonies, the test and/or medium of the invention may also be used for detecting non-fermenting bacteria, in particular in case the medium comprises nutrients for supporting the growth of such bacteria.

In an embodiment, the invention provides a test, medium and/or solution comprising at least two different aminoglycoside antibiotics and nutrients for supporting the growth of Gram negative bacteria, in particular *Enterobacteriaceae.*

The term "comprising", for the purpose of the present specification, is intended to mean "comprises, amongst other". It is not intended to mean "consists only of".

The test, medium and/or solution preferably comprises nutrients for supporting the growth of the microorganisms that are to be selected. The culture medium preferably comprises a carbon source, in particular carbohydrates, such as poly-, oligo-, di- and/or monosaccharides that can be metabolized by the bacteria to be selected. Preferably, the culture medium comprises di- and/or monosaccharides. In an embodiment, the culture medium may contain one or more selected from sucrose, glucose and lactose.

The test, medium and/or solution preferably comprises other nutrients and or factors for supporting bacterial growth, such as proteins, peptides, amino acids, and/or salts needed for bacterial growth. For example, the invention test, medium and/or solution may comprise Müller-Hinton Broth (MHB) and/or Eosine Methylene Blue (EMB) Culture Medium.

In an embodiment of the invention, said at least two different aminoglycoside antibiotics comprise at least one 4,6-disubstituted deoxystreptamine aminoglycoside, preferably at least two 4,6-disubstituted deoxystreptamine aminoglycosides.

In an embodiment of the invention, said at least two different aminoglycoside antibiotics comprise a first antibiotic selected from 4,6-disubstituted deoxystreptamine aminoglycosides, and a second antibiotic selected from the group consisting of: 4,6-disubstituted deoxystreptamine aminoglycosides, 4,5-disubstituted deoxystreptamine aminoglycosides, and non-deoxystreptamine aminoglycoside.

In an embodiment of the invention, said at least two different aminoglycoside antibiotics comprise amikacin and gentamicin.

In an embodiment of the invention, said medium and/or solution comprises, independently, 0.1 to 100 mg/L, preferably 1 to 90 mg/L, more preferably 4 to 90 mg/L, and most preferably 10 to 80 mg/L of each one of said at least two aminoglycoside antibiotics. In other words, each of said at least two different aminoglycoside antibiotics is independently present at the aforementioned concentrations.

In a preferred embodiment of the invention, said medium and/or solution comprises, independently, 15 to 70 mg/L, preferably 20 to 50 mg/L, and most preferably 25 to 40 mg/L of each one of said at least two aminoglycoside antibiotics.

In an embodiment, the method of the invention comprises the step of adjusting the pH of the test, medium and/or solution to a desired value, for example by adding drops of acid or base as appropriate. In some embodiments, the pH of a stock solution may be adjusted, as described elsewhere in this specification.

The method of the invention preferably comprises the step of inoculating said test, medium and/or solution with a sample comprising bacteria. The sample is preferably suspected of comprising bacteria. It is supposed that said sample comprises bacteria and the test is conducted for testing the occurrence of a resistance as specified elsewhere in the specification. Of course, the invention does not exclude the possibility of running the method of the invention with a sample that lacks bacteria. A sample may be actually devoid of bacteria. For example, the sample may be consciously devoid of bacteria for the purpose of providing a negative control. One may also envisage that the sample may stem of an individual suspected to have a bacterial infection, but that the individual does actually not have an infection as suspected. In such a case, a negative test outcome will be generally obtained.

The sample may be a body fluid or derived from a body fluid. In some embodiments, the samples may be derived from blood, serum, urine, saliva and/or stool. Preferably, the sample is a biological sample taken previously and/or in a separate step from an individual. In some embodiments, the sample is pretreated, for example for concentrating bacteria. For example, the sample may be a pellet of a centrifuged urine sample, or be a sample obtained from culturing bacteria contained in a body fluid sample, for example a blood culture sample, or a sample obtained from culturing bacteria on a solid or liquid medium, for example.

In an embodiment of the invention, said test, medium and/or solution preferably comprises comprises a color indicator. Preferably, a color of said color indicator is pH dependent. For example, said color indicator is phenol red.

Preferably, said test, medium and/or solution comprises nutrients, wherein, after inoculation, metabolic activity of said bacteria results in a change of the pH of the test, medium and/or solution, thereby changing the color of said color indicator.

In a preferred embodiment of said test, medium and/or solution, said nutrients comprise a carbohydrate source, and wherein metabolic activity of Gram negative bacteria, such as *Enterobacteriaceae,* exhibiting 16S rRNA methylase activity results in acidification of the solution due to metabolism of said carbohydrate source due to said metabolic activity. For example, if phenol red is used as a color indicator, a color change from orange to yellow is detected after incubation in presence of metabolic activity due to the pH decrease.

Preferably, the method of the invention comprises the step of incubating the medium, solution and/or test after inoculation, preferably at conditions (e.g. temperature, humidity) that are conducive for bacterial growth, in particular growth of Gram negative, aerobic and/or facultative anaerobic bacteria.

The method of the invention preferably comprises the step of screening for bacterial growth and/or metabolic activity in said medium and/or solution after incubation. In some embodiments, said screening for bacterial growth and/or metabolic activity is conducted in 3 hours or less starting from inoculation, preferably in 2.5 hours or less and most preferably in 2 hours or less starting from inoculation of the sample.

In an embodiment of the invention, said test outcome is determined in 3 hours or less starting from the inoculation of the medium and/or solution, and on the basis of the color of the medium and/or solution, wherein the test outcome is positive if the color indicator indicates a change of the acidity of the medium and/or solution. In a preferred embodiment, the change of acidity is a decrease of the pH and/or an acidification of the medium and/or solution. Preferably, if a carbohydrate source is present in the test, medium and/or solution, the metabolism of the carbohydrate results in a decrease of the pH.

The method of the invention preferably comprises finding a positive test outcome indicating said multiple aminoglycoside resistance due to rRNA methylase if bacterial growth and/or metabolic activity is detected. Said bacterial growth and/or metabolic activity is preferably determined indirectly, via the color of a color indicator. A change of color resulting from a pH change due to bacterial metabolic activity is thus preferably interpreted as the presence of bacterial growth and/or metabolic activity. Preferably, the method, kit, solution and/or medium allows detecting multiple aminoglycoside resistance in fermenting *Enterobacteriaceae.*

A test the read out of which is based on a color change due to pH change, which in turn is due to metabolism of a carbohydrate source such as glucose or lactose, for example, is preferably suitable to detect resistance in fermenting bacteria, in particular fermenting *Enterobacteriaceae.*

In accordance with the invention, said medium may be a solid or a liquid medium. For example, the medium may be in the form of an aqueous solution and/or suspension.

In some embodiments, the present invention preferably provides a test kit and/or solution for conducting the method of the invention. The test kit may comprise separate components, that may be mixed together by a user for providing said a medium and/or solution. For example, the test kit comprises one or more solution comprising the at least two aminoglycoside antibiotics. Said test kit may comprise a solution comprising a soluble carbohydrate selected from sucrose, glucose and lactose, preferably glucose. The soluble carbohydrate may be present in the same solution as said aminoglycoside antibiotics or may be present in a separate solution of the test kit. In an embodiment, if the color indicator is phenol red, the test kit may comprise a powder of phenol red or may comprise a solution comprising phenol red. The solution and/or medium for inoculation with bacteria may preferably be obtained by mixing the various components of the kit.

In a preferred embodiment, the test of the invention comprises a first or stock solution comprising said at least two aminoglycoside antibiotics and a second or reaction solution comprising nutrients and/or said color indicator. Preferably, the second or reaction solution comprises a source of carbohydrates and said color indicator. More preferably, the second or reaction solution comprises glucose and said color indicator. Preferably, the second or reaction solution further comprises additional factors and/or nutrients supportive of growth of *Enterobacteriaceae.* In a preferred embodiment, the second or reaction solution comprises Müller-Hinton Broth (MHB)-powder, preferably cation adjusted MHB-powder.

Preferably, a pH of said second or reaction solution is adjusted with acid or base. In an embodiment, the pH is adjusted to a pH value in the range of 6.5 - 7.0, for example 6.6 - 6.9.

In an embodiment, the test, medium and/or solution is provided in a liquid form, and the test enables rapid evaluation of the presence of resistance in accordance with the invention, for example within 3 hours or less following incubation.

Preferably, if the rapid test is based on the basis of a color change due to change of the acidity of the inoculated solution, the test is preferably not performed with bacterial colonies grown directly before on acidifying media such as Drigalski, Mac Conkey or Bromocresol purple (BCP) culture media. Preferably, the bacteria used for inoculation were not grown on an acidifying medium before inoculation, or, if so, are separated from such acidifying medium and washed before inoculation.

The present invention preferably provides a comparatively rapid test, allowing a read-out within several hours, in particular less than 12 hours, for example within 3 or less hours. In a preferred embodiment, the rapid test is based on non-solid and/or liquid medium, for example a suspension or solution as described elsewhere in this specification. The rapid test is preferably adapted to detecting specifically *Enterobacteriaceae* exhibiting said rRNA methylase activity. The rapid test comprises a carbohydrate source, such as glucose, that acidifies the medium in presence of metabolism by the bacteria exhibiting resistance traits to be selected. In addition to a carbohydrate source that can be metabolized by *Enterobacteriaceae,* the test may comprise factors that are required for enterobacterial growth.

In an embodiment, the comparatively rapid test and the corresponding solution and/or medium is not appropriate or suitable for detecting non-fermenters, such as *Acinetobacter baumannii* and *Pseudomonas aeruginosa* exhibiting said rRNA methylase activity.

In an embodiment, the invention provides a solid culture medium, for example an agar-based culture medium for selecting bacteria having the resistance traits as detailed elsewhere in this specification. In the case of a solid culture medium, a test outcome is preferably determined by assessing the formation of bacterial colonies, which can generally be done after longer periods than just 3 hours. Generally, the presence of bacterial colonies can be determined by visual inspection as from about 24 hours after inoculation. Preferably said medium is a solid medium and the test outcome is positive if growing colonies are detectable on the medium within 24 to 48 hours.

In an embodiment, the medium of the invention comprises, in addition to said at least two different aminoglycoside antibiotics, a daptomycin antibiotic and/or an antifungal agent.

In an embodiment, the medium of the invention further comprises, in addition to said at least two different aminoglycoside antibiotics, a daptomycin antibiotic and/or an antifungal agent. Preferably, said daptomycin antibiotic is selected from daptomycin and analogues thereof, such as vancomycin, preferably it is daptomycin.

In an embodiment, the concentration of said daptomycin antibiotic in said medium and/or solution is 0.5 to 50 µg/ml, preferably 1 to 30 µg/ml, more preferably 5 to 15 µg/ml.

In an embodiment, said antifungal agent is amphotericin B.

In an embodiment, the concentration of said antifungal agent is 0.1 to 50 µg/ml, preferably 0.5 to 25 µg/ml, more preferably 1 to 10 µg/ml.

In an embodiment, the medium contains lactose as a source of carbohydrates and one or more selected from amino acids, peptides and/or proteins. For example, the medium may comprise peptone.

In an embodiment, said medium comprises Eosine Methylene Blue (EMB). Preferably, the medium comprises EMB Culture Medium, preferably based on agar. Preferably the medium according to the formulation described by M. Levine in 1918 (J Infect Dis 23: 43-47) is used. The EMB medium is a selective-differential plating medium for the detection and isolation of gram-negative bacteria.

In a preferred embodiment, the culture medium, in particular the solid culture medium is also suitable to detect non-fermenters exhibiting the rRNA methylase activity. Preferably, the culture medium according to this embodiment comprises nutrients that allows growth on non-fermenters. Preferably, the culture medium allows selecting for non-fermenters such as *Pseudomonas aeruginosa* and *Acinetobacter baumannii,* exhibiting the methylase-mediated resistance to aminoglycoside antibiotics.

Preferably, the read-out of a test comprising a solid culture medium is based on visually determining the presence of bacterial colonies. In a preferred embodiment, the read out, in terms of resistance traits, of a test based on a solid culture medium is not or not only based on a color change of the medium.

While certain of the preferred embodiments of the present invention have been described and specifically exemplified above, it is not intended that the invention be limited to such embodiments. Various modifications may be made thereto without departing from the scope and spirit of the present invention, as set forth in the following claims. Herein below, examples of the invention are disclosed. These examples are for illustration only and are not intended to limit the scope of the present invention.

### Examples

### Example 1: Rapid Aminoglycoside NP test

### Isolates collection

A total of 48 isolates from clinical samples of worldwide origin were used to evaluate the performance of the Rapid Aminoglycoside NP (Nordmann/Poirel) test, including 38 isolates resistant to AG and 10 isolates susceptible to AG (Table 1). The AG resistant isolates were well characterized at the molecular level for their resistance mechanisms with 18 strains harboring various 16S rRNA methylases and 20 strains expressing different AG-modifying enzymes (acetyltransferases (AAC), nucleotidyltransferases (ANT), and phosphotransferases (APH)) (Table 1).

### Minimum Inhibitory Concentrations (MICs) determination

MICs of amikacin and gentamicin were determined using the broth microdilution method in cation-adjusted Mueller-Hinton broth, as recommended by the Clinical Laboratory Standard Institute (CLSI) guidelines (8). Amikacin and gentamicin sulfate (Fluorochem, Dertbyshire, UK) were tested over a range of dilutions from 0.25 to 256 µg/ml. A final inoculum of 5.10⁵ CFU/ml of each strain was distributed in the 96 wells tray (Sarstedt, Nümbrecht, Germany). All experiments were repeated in triplicate and *E. coli* ATCC 25922 was used as quality control. According to the CLSI breakpoints (9), Enterobacteriaceae isolates with gentamicin MICs ≤ 4 and amikacin MICs ≤ 16 mg/L were categorized as susceptible, while those with gentamicin MICs ≥ 16 and amikacin MICs ≥ 64 mg/L were categorized as resistant.

### Rapid Aminoglycoside NP test

### (i) Reagents and solutions

### Stock solutions of AG

Amikacin and gentamicin (Fluorochem, Dertbyshire, UK) were diluted into water to obtain stock solution at a concentration of 1.6 mg/ml. The antibiotic powders and the stock solutions can be stored at 4°C and -20°C, respectively, before their use.

### Rapid Aminoglycoside NP solution

To prepare 250 ml of the Rapid Aminoglycoside NP solution, the culture medium and the pH indicator were mixed in a glass bottle as follows: 6.25 g of MHB-CA powder, 0.0125 g of phenol red (Sigma Aldrich), and 225 ml of distilled water. The pH of the solution was adjusted precisely to 6.7 by adding drops of 1 M HCl. This solution was then autoclaved at 121°C for 15 min. After cooling the solution to room temperature, 25 ml of D (+)-glucose anhydre 10 % (Roth, Karlsruhe, Germany) sterilized by filtration, was added. The final concentrations in the Rapid Aminoglycoside NP solution were consequently 2.5 % of MHB-CA powder, 0.005 % of phenol red indicator, and 1 % of D (+)-glucose. This Rapid Aminoglycoside NP solution can be kept at 4°C for one week or at -20°C for one year. Noteworthy, this solution has to be pre-warmed at 37°C before its use to prevent growth delay and therefore a delay for obtaining the color change of the test.

### AG-containing Rapid Aminoglycoside NP solution

Amikacin and gentamicin were added extemporaneously to the Rapid Aminoglycoside NP solution and mixed into sterile tubes to obtain a Rapid Aminoglycoside NP solution containing a final concentration of amikacin (40 µg/ml) plus gentamicin (40 µg/ml). As an example, 25 µl of the stock solution at 1.6 mg/ml was added to 1 ml of Rapid Aminoglycoside NP test solution for testing one single isolate, and respective negative and positive controls.

### (ii) Bacterial inoculum

A standardized Enterobacteriaceae inoculum was prepared using freshly-obtained (overnight) bacterial colonies grown on Luria-Bertani or Mueller-Hinton plates. The bacterial colonies were resuspended into 10 ml of sterile NaCl 0.9 % to obtain a 3 to 3.5 McFarland optical density (ca. 10⁹ CFU/ml). This corresponds to ca. a single 10-µl full loop of bacterial colonies diluted in 10 ml of NaCl. A bacterial suspension was prepared for each isolate to be tested, and for the susceptible and resistant isolates used as controls (isolates FR136 and R989 respectively in Table 1). As recommended by the EUCAST guidelines for susceptibility testing, the bacterial suspensions were used within 15 min, without exceeding 60 min after their preparation.

### (iii) Tray inoculation

The test was performed in a 96-well polystyrene microtest plate (round base, with lid, sterile, ref. 82.1582.001, Sarstedt, Nümbrecht, Germany). For each isolate, two wells are inoculated in parallel with the bacterial suspension, respectively with or without the AG mix. The Rapid Aminoglycoside NP test was performed as follows (Figure):
Step1: 150 µl of AG-free solution was transferred in the four wells of the first line.
Step 2: 150 µl of the Rapid Aminoglycoside NP solution containing the AG mix was transferred in the four wells of the second line.
Step 3: 50 µl of NaCl 0.9 % was added in the two wells of the first column.
Step 4: 50 µl of the AG-susceptible isolate suspension used as negative control (*E. coli* ATCC 25922) was added in the two wells of the second column.
Step 5: 50 µl of the AG-resistant isolate suspension used as positive control (16S rRNA methylase producer) was added in the two wells of the third column.
Step 6: 50 µl of the tested isolate suspension was added in the two wells of the last column. After mixing the bacterial suspension to the reactive medium by pipetting up and down, the final concentration of bacteria was ca. 10⁸ CFU/ml in each well and the final concentrations of amikacin and gentamicin were 30 µg/ml each.

### (iv) Tray incubation

The inoculated tray was incubated up to 4 h at 35±2°C in ambient air, not sealed and without agitation. Non sealing of the tray was done due to the oxygen requirement for glucose metabolism of *Enterobacteriaceae.*

### (v) Tray reading

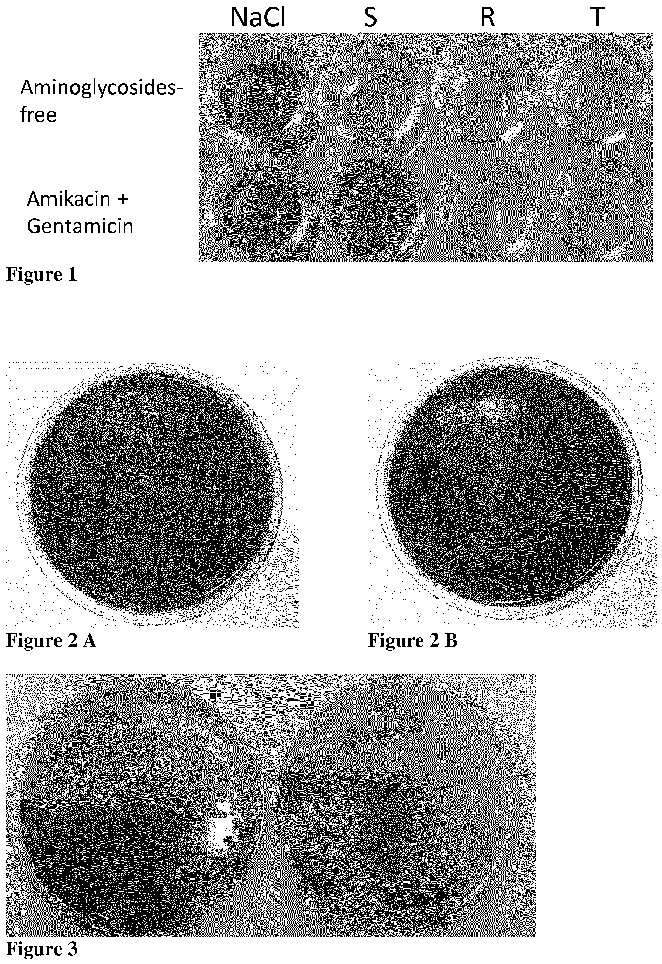

Visual inspection of the tray was made after 10 min (checking for no spontaneous color change), and then every hour during three hours. The test was considered as positive (multiple AG resistance) if the isolate grew in presence of the AG mix while it was considered as negative (AG susceptibility) if the susceptible isolate did not grow in presence of the AG mix (Figure 1). The test was considered as interpretable if: (i) both wells with NaCl 0.9% without bacterial suspension (first column wells) remained orange (absence of medium contamination), (ii) the wells with bacterial suspension and AG-free (wells of the first line except the first well that contained NaCl) turned from orange to yellow confirming the metabolism of glucose by the isolates, (iii) the wells with the susceptible reference bacterial suspension (negative control) gave negative result (second column wells), and (iv) the wells with the resistant reference bacterial suspension (positive control) gave positive result (third column wells), as explained hereafter. The test was positive when the well containing the AG mix and the isolate to be tested turned from orange to yellow, giving exactly the same color than the well without AG indicating a glucose metabolism and growth in presence of the AG mix, i.e. AG resistance (Figure 1). The test was considered as negative when the well containing the AG mix with the isolate to be tested remained orange (unchanged color) (Figure 1). Results were interpreted blindly by a technician who did not know which were the resistant and susceptible isolates.

**Figure 1** shows representative results of the Rapid Aminoglycoside NP (Nordmann/Poirel) test. The Rapid Aminoglycoside NP test was performed with a reference susceptible isolate (S) in the second column and with a reference 16S rRNA methylase isolate (R) in the third column, in a reaction medium without (upper line) and with (lower line) the AG mix (amikacin + gentamicin). Non inoculated wells are shown in the first column (NaCl-containing wells). The tested isolate (T) in the fourth column grew in the presence of the mix of aminoglycosides and presented therefore a multiple resistance to aminoglycosides. The photograph was taken after a 1-h incubation time.

### Other tested conditions

The Rapid Aminoglycoside NP test was also performed by testing other concentrations of the same mix of AG molecules or other AG mix such as tobramycin, and kanamycin or amikacin and kanamycin. The test was performed also after an overnight culture on different agar plates to determine the potential impact of the culture medium prior to the realization of the test. The following culture media were tested: non-selective culture medium such Columbia agar + 5% sheep blood (bioMérieux), the non-selective chromogenic medium UriSelect 4 (Bio-Rad), chocolate agar + PolyVitex (bioMérieux), Eosine methylene blue agar (Sigma Aldrich), Drigalski agar (Bio-Rad), MacConkey agar (*VWR BDH Prolabo, Leuven, Belgium*) and bromocresol purple (bioMérieux).

### Results and Conclusion

A total of 48 Enterobacteriaceae isolates were tested to evaluate the performance of the Rapid Aminoglycoside NP test, including 38 AG resistant isolates and 10 AG susceptible isolates (Table 1). Among the 38 AG resistant isolates, 18 isolates produced 16S rRNA methylases and presented high MIC values for both amikacin and gentamicin, while 20 produced AG-modifying enzymes and exhibited various level of resistance for amikacin and gentamicin (Table 1).

An excellent correlation was obtained between multiple AG resistance and positivity of the test and conversely, non-multiple AG resistance and negativity of the test (Table 1). The sensitivity and the specificity of the test were excellent (100%), compared with broth dilution taken as the gold standard.

The reading of the color change of the wells every hour revealed that final results were obtained 2 h after incubation when the tray was incubated at 35±2°C under an ambient atmosphere. AG susceptibility results obtained with the Rapid Aminoglycoside NP test were consequently obtained at least 16 h sooner than with disk diffusion method, E-test system or broth dilution method.

Solutions containing other combinations of AG or other AG molecules did not modify the results. The test may be performed from cultured bacteria grown on media such as Luria Bertani, Mueller-Hinton, Uriselect-4, eosine-methylene blue medium, blood agar, and chocolate agar. Interference may be observed with colonies grown on acidifying culture media such as Drigalski, Mac Conkey and bromocresol purple. The test has been optimized in its present form and other conditions (pH indicator change, non-cation-adjusted culture medium, inoculum size, glucose and pH indicator concentrations, other polystyrene-containing trays...) were tested and gave less optimal results.

**Table 1: Rapid Aminoglycoside NP test results for AG-resistant Enterobacteriaceael isolates producers of 16S rRNA methylases and producers of AG-modifying enzymes, and for AG-susceptible Enterobacteriaceae isolates.**

| Isolate | Species | Mechanism of AG resistance | Amikacin | | Gentamicin | | RANP result^{b} |
|---|---|---|---|---|---|---|---|
| | | | MIC^{a} | Pheno type | MIC^{a} | Pheno type | |
| AG susceptible isolates | | | | | | | |
| FR136 | *E. coli* ATCC 25922 | - | 2 | S | 0.5 | S | - |
| R2278 | *E. coli* ATCC 35218 | - | 4 | S | 1 | S | - |
| R1436 | *E. coli* | - | 2 | S | 1 | S | - |
| R1438 | *E. coli* | - | 2 | S | 1 | S | - |
| R347 | *K. pneumoniae* | - | 4 | S | 0.5 | S | - |
| S155 | *K. pneumoniae* | - | 2 | S | 0.5 | S | - |
| R1433 | *E. cloacae* | - | 4 | S | 2 | S | - |
| R713 | *E. cloacae* | - | 4 | S | 0.5 | S | - |
| HM | *E. cloacae* | - | 4 | S | 2 | S | - |
| R2260 | *P. mirabilis* | - | 4 | S | 4 | S | - |

| AG resistant isolates producers of methylases | | | | | | | |
|---|---|---|---|---|---|---|---|
| R989 | *E. coli* | *armA* | >256 | R | >256 | R | + |
| R1012 | *E. coli* | *armA* | >256 | R | >256 | R | + |
| R500 | *K. pneumoniae* | *armA* | >256 | R | >256 | R | + |
| R108 | *K. pneumoniae* | *armA* | >256 | R | >256 | R | + |
| R2257 | *K. pneumoniae* | *armA* | >256 | R | >256 | R | + |
| R2258 | *E. cloacae* | *armA* | >256 | R | >256 | R | + |
| 62Carb | *E. coli* | *rmtB* | >256 | R | >256 | R | + |
| E28 | *E. coli* | *rmtB* | >256 | R | >256 | R | + |
| R107 | *E. coli* | *rmtB* | >256 | R | >256 | R | + |
| R990 | *E. coli* | *rmtB* | >256 | R | >256 | R | + |
| R262 | *E. coli* | *rmtC* | >256 | R | >256 | R | + |
| R451 | *E. coli* | *rmtC* | >256 | R | >256 | R | + |
| R265 | *P. stuartii* | *rmtC* | >256 | R | >256 | R | + |
| R38 | *P. stuartii* | *rmtC* | >256 | R | >256 | R | + |
| N34 | *K. pneumoniae* | *rmtC* | >256 | R | >256 | R | + |
| R502 | *K. pneumoniae* | *rmtF* | >256 | R | >256 | R | + |
| R2160 | *K. pneumoniae* | *rmtG* | >256 | R | >256 | R | + |
| R4100 | *E. coli TOP10* | *npmA* | 128 | R | 64 | R | + |
| | | | | | | | |

| AG resistant isolates producers of AG-modifying enzymes | | | | | | | |
|---|---|---|---|---|---|---|---|
| pWP701 | *E. coli* | AAC(3)-IV | 1 | S | 128 | R | - |
| pFCT4392 | *E. coli* | AAC(3)-Ia | 1 | S | 32 | R | - |
| 390 | *E. coli* | AAC(3)-IV | 16 | S | 128 | R | - |
| 22089 | *E. coli* | AAC(3)-I + AAC(3)-V | 16 | S | 128 | R | - |
| 97 | *E. coli* | AAC(3)-IV | 2 | S | 32 | R | - |
| 92/31 | *E. coli* | AAC(3)-IV | 4 | S | 16 | R | - |
| 4000 | *E. coli* | APH(3')-I + AAC(3)-V | 4 | S | 64 | R | - |
| pFCT1163 | *E. coli* | AAC(6')-Ia | 128 | R | 4 | S | - |
| 1054 | *E. coli* | AAC(6')-I | 8 | S | 8 | I | - |
| 1488 | *E. coli* | AAC(6')-II | 4 | S | 16 | R | - |
| 1054 | *E. coli* | AAC(6')-I | 16 | S | 8 | I | - |
| RP4 | *E. coli* | APH(3')-Ib | 1 | S | 1 | S | - |
| 122971 | *E. coli* | ANT(2") | 4 | S | 128 | R | - |
| 1800 | *K. pneumoniae* | AAC(6')-I + AAC(3)-I | 32 | I | 16 | R | - |
| 13000 | *K. pneumoniae* | APH(3')-I + AAC(3)-V | 0.5 | S | 128 | R | - |
| 22233 | *K. pneumoniae* | AAC(3)-V | 16 | S | 128 | R | - |
| 1110 | *E. cloacae* | ANT(2") | 1 | S | 32 | R | - |
| BM2667 | *P. stuartii* | APH(3')-VI + AAC(2') | 64 | R | 8 | S | - |
| 531 | *P. rettgeri* | AAC(2') | 16 | S | 4 | S | - |
| 4290 | *S. marcescens* | AAC(3)-I | 4 | S | 64 | R | - |

| | | | | | | | |
|---|---|---|---|---|---|---|---|
| AG, aminoglycosides; AAC, N-Acetyltransferases; ANT, O-Adenyltransferases, APH, O-Phosphotransferases; MIC, Minimum inhibitory concentrations; RANP, Rapid Aminoglycoside NP test. ^{a}MICs were determined by broth microdilution method. ^{b}Results of the RANP test after 2 hours of incubation at 35±2°C under ambient air. | | | | | | | |

Complete species name of strains in Table 1: *Escherichia coli, Klebsiella pneumoniae, Enterobacter cloacae, Providencia stuartii, Providencia rettgeri, Serratia marcescens, Proteus mirabilis.*

The study has at least two limitations. First, the positivity of the test does not always superimpose the presence of 16S rRNA methylases. Combined expression of nucleotidyl, phosphoryl and acetyltransferases in a given Enterobacteriaceae strain may indeed confer resistance to both gentamicin and amikacin. Second, this test has not been evaluated with non-fermenters such as *Pseudomonas aeruginosa* and *Acinetobacter baumannii,* that may also express 16S rRNA methylases (10,11).

The Rapid Aminoglycoside NP test may be implemented for determining multiple resistance to AG in particular in countries facing an endemic spread of carbapenemase producers (1). The use of the Rapid Aminoglycoside NP test may also contribute to rapidly identify carriers of MDR isolates expressing plasmid-mediated 16S rRNA methylases to a rapid implementation of hygiene measures and to control their spread. In a world that is moving rapidly toward pandrug resistance, the Rapid Aminoglycoside test may guide prescription of novel AG such as plazomicin by differentiating aminoglycoside modifying enzymes and methylase producers. Plazomicin is indeed active against aminoglycoside modifying enzymes such as the nucleotidyl, acetyl and phosphoryl enzymes (12), while plazomicin is inactive against 16S rRNA methylase producers (13). This test may also support the development of such novel AG molecule facilitating patient enrollment in pivotal clinical trials.

The Rapid Aminoglycoside NP test is easy-to-perform, rapid (< 2 h), sensitive, and specific. It detects multiple AG resistance from bacterial cultures from infected samples or from selective media prior to obtaining any antibiotic susceptibility testing results.

### Example 2: Selective culture medium for screening multiple aminoglycoside resistant bacteria

### Materials and Methods

The optimal screening medium retained was based on the Eosine-Methylene-Blue (EMB) medium (product n°70186;EMB Fluka, Saint-Louis, MO, USA) that is selective for gram negatives. This medium may also contribute to species identification by differentiating lactose fermenters (black colonies) from non-fermenters (colorless or light lavender). The optimal final concentration of amikacin and gentamicin (Sigma Aldrich, Saint-Louis, MO, USA) were 30 µg/ml each. Daptomycin (Novartis, Horsham, United Kingdom) was added at a concentration of 10 µg/ml. Amphotericin B (Bristol-Myers-Squibb, Rueil-Malmaison, France) was also added as anti-fungi molecule at a concentration of 5 µg/ml.

The stock solutions of amikacin, gentamicin, daptomycin, and amphotericin B and instruction for use of the SuperAminoglycoside medium are indicated in Table 2. Those stock solutions were prepared and may be kept at -20°C during 1 year. The diluted powder of EMB was autoclaved at 121°C for 15 min. After cooling the EMB medium for one hour at 56°C, the antibiotic stock solutions were added. Poured plates were stored at 4°C and protected from direct light exposure up to one week.

**Table 2: Preparation of the selective medium**

| Compound | Stock solution (mg/ml) | Quantity or volume to add^{a} | Final concentration^{b} |
|---|---|---|---|
| EMB agar powder | - | 15 g | 3.75 % |
| Distilled water | - | 400 ml | |
| Gentamicin | 30 | 400 ul | 30 |
| Amikacin | 30 | 400 µl | 30 |
| | | | |
| Daptomycin | 20 in water | 200 µl | 10 |
| Amphotericin B | 2 in D(+)-glucose 10 % | 100 µl | 5 |

| | | | |
|---|---|---|---|
| ^{a} The volume of 400 ml of medium was for i.e. twenty plates. ^{b} Concentration are in micrograms per milliliter unless noted otherwise. | | | |

A total of 59 isolates were included in the study to evaluate the performance of the SuperAminoglycoside medium that included 20 methylase producers of worldwide worldwide origin (Table 3). Twelve strains were susceptible to aminoglycoses while twenty seven strains produced aminoglycoside-modifying enzymes. *Enterobacteriaceae, Pseudomonas aeruginosa* and *Acinetobacter baumannii* isolates were included in the study (Table 3). The AG resistant isolates were characterized at the molecular level for their resistance mechanism with 16S rRNA methylases and AG-modifying enzymes (acetyl, nucleotidyl, and phosphoryl enzymes) (Table 3).

MICs of amikacin and gentamicin were determined as described above in Example 1.

**Table 3: MICs of amikacin and gentamicin for the studied strains and limit of detection of the aminoglycoside-containing culture medium EMB 30/30**

| Strain | Species | Resistance | MIC (µg/ml)^{b} | | L. d. l. (CFU/ml)^{c} | L. d. l. in stool (CFU/ml)^{c} |
|---|---|---|---|---|---|---|
| | | | Amik acin | Genta micin | | |
| Susceptible strains | | | | | | |
| R2298 | *E. coli* ATCC 25922 | - | 2 | 1 | > 1 x 10⁸ | > 1 x 10⁸ |
| R2278 | *E. coli* ATCC 35218 | - | 4 | 1 | > 1 x 10⁸ | > 1 x 10⁸ |
| R1436 | *E. coli* | - | 2 | 1 | > 1 x 10⁸ | > 1 x 10⁸ |
| R1438 | *E. coli* | - | 2 | 1 | > 1 x 10⁸ | > 1 x 10⁸ |
| R347 | *K. pneumoniae* | - | 4 | 0.5 | > 1 x 10⁸ | > 1 x 10⁸ |
| S155 | *K. pneumoniae* | - | 2 | 0.5 | > 1 x 10⁸ | > 1 x 10⁸ |
| R1433 | *E. cloacae* | - | 4 | 2 | > 1 x 10⁸ | > 1 x 10⁸ |
| R713 | *E. cloacae* | - | 4 | 0.5 | > 1 x 10⁸ | > 1 x 10⁸ |
| HM | *E. cloacae* | - | 4 | 2 | > 1 x 10⁸ | > 1 x 10⁸ |
| R2260 | *P. mirabilis* | - | 4 | 4 | > 1 x 10⁸ | > 1 x 10⁸ |
| R1395 | *A. baumannii* | - | 2 | 1 | > 1 x 10⁸ | > 1 x 10⁸ |
| R191 | *P. aeruginosa* | - | 8 | 2 | > 1 x 10⁸ | > 1 x 10⁸ |

| Methylase-producing strains | | | | | | |
|---|---|---|---|---|---|---|
| R4100 | *E. coli* | npmA | 258 | 258 | 2 x 102 | 2 x 102 |
| R989 | *E. coli* | armA | >512 | >256 | 2 x 10¹ | 1 x 10² |
| R1012 | *E. coli* | armA | >512 | >256 | 1 x 10¹ | 3 x 10¹ |
| 62Carb | *E. coli* | rmtB | >512 | >256 | 2 x 10² | 2 x 10² |
| E28 | *E. coli* | rmtB | >512 | >256 | 4 x 10¹ | 4 x 10¹ |
| R107 | *E. coli* | rmtB | >512 | >256 | 2 x 10¹ | 2 x 10¹ |
| R990 | *E. coli* | rmtB | >512 | >256 | 2 x 10¹ | 1 x 10¹ |
| R262 | *E. coli* | rmtC | >512 | >256 | 3 x 10² | 3 x 10² |
| R451 | *E. coli* | rmtC | >512 | >256 | 3 x 10¹ | 2 x 10¹ |
| R500 | *K. pneumoniae* | armA | >512 | >256 | 2 x 10² | 2 x 10² |
| R108 | *K. pneumoniae* | armA | >512 | >256 | 4 x 10¹ | 2 x 10¹ |
| R2257 | *K. pneumoniae* | armA | >512 | >256 | 1 x 10¹ | 2 x 10¹ |
| N34 | *K. pneumoniae* | rmtC | >512 | >256 | 3 x 10¹ | 1 x 10² |
| R502 | *K. pneumoniae* | rmtF | >512 | >256 | 2 x 10² | 3 x 10² |
| R2160 | *K. pneumoniae* | rmtG | >512 | >256 | 3 x 10² | 3 x 10² |
| R2258 | *E. cloacae* | armA | >512 | >256 | 2 x 10¹ | 2 x 10¹ |
| R265 | *P. stuartii* | rmtC | >512 | >256 | 3 x 10² | 3 x 10² |
| R38 | *P. stuartii* | rmtC | >512 | >256 | 2 x 10¹ | 1 x 10¹ |
| R263 | *P. mirabilis* | armA | >512 | >256 | 4 x 10² | 3 x 10² |
| R264 | *A. baumannii* | armA | >512 | >256 | 3 x 10² | 3 x 10² |

| Aminoglycoside resistant and non-methylase producing strains | | | | | | |
|---|---|---|---|---|---|---|
| pWP701 | *E. coli* | AAC(3)-IV | 1 | 128 | 9 x 10⁷ | > 1 x 10⁸ |
| pFCT4392 | *E. coli* | AAC(3)-Ia | 1 | 32 | 3 x 10⁷ | > 1 x 10⁸ |
| 390 | *E. coli* | AAC(3)-IV | 16 | 128 | > 1 x 10⁸ | > 1 x 10⁸ |
| 22089 | *E. coli* | AAC(3)-I + AAC(3)-V | 16 | 128 | > 1 x 10⁸ | > 1 x 10⁸ |
| 97 | *E. coli* | AAC(3)-IV | 2 | 32 | > 1 x 10⁸ | > 1 x 10⁸ |
| 92/31 | *E. coli* | AAC(3)-IV | 4 | 16 | > 1 x 10⁸ | > 1 x 10⁸ |
| 4000 | *E. coli* | APH(3')-I + AAC(3)-V | 4 | 64 | > 1 x 10⁸ | > 1 x 10⁸ |
| pFCT1163 | *E. coli* | AAC(6')-Ia | 128 | 4 | 1 x 10⁸ | > 1 x 10⁸ |
| 1054a | *E. coli* | AAC(6')-I | 8 | 8 | > 1 x 10⁸ | > 1 x 10⁸ |
| 1488 | *E. coli* | AAC(6')-II | 4 | 16 | > 1 x 10⁸ | > 1 x 10⁸ |
| 1054b | *E. coli* | AAC(6')-I | 16 | 8 | > 1 x 10⁸ | > 1 x 10⁸ |
| RP4 | *E. coli* | APH(3')-Ib | 1 | 2 | 7 x 10⁷ | > 1 x 10⁸ |
| 122971 | *E. coli* | ANT(2") | 4 | 128 | > 1 x 10⁸ | > 1 x 10⁸ |
| 1800 | *K. pneumoniae* | AAC(6')-I + AAC(3)-I | 32 | 16 | 1 x 10⁸ | > 1 x 10⁸ |
| 13000 | *K. pneumoniae* | APH(3')-I + AAC(3)-V | 0.5 | 128 | > 1 x 10⁸ | > 1 x 10⁸ |
| 22233 | *K. pneumoniae* | AAC(3)-V | 16 | 128 | > 1 x 10⁸ | > 1 x 10⁸ |
| 1110 | *E. cloacae* | ANT(2") | 1 | 32 | > 1 x 10⁸ | > 1 x 10⁸ |
| BM2667 | *P. stuartii* | APH(3')-VI + AAC(2') | 64 | 16 | 1 x 10⁸ | > 1 x 10⁸ |
| 531 | *P. rettgeri* | AAC(2') | 16 | 4 | 6 x 10⁷ | > 1 x 10⁸ |
| 4290 | *S. marcescens* | AAC(3)-I | 4 | 64 | 4 x 10⁷ | > 1 x 10⁸ |
| BM2580 | *A. baumannii* | APH(3')-VI | 64 | 2 | > 1 x 10⁸ | > 1 x 10⁸ |
| 8541 | *A. baumannii* | APH(3')-VI | 128 | 0.5 | > 1 x 10⁸ | > 1 x 10⁸ |
| 4996 | *A. baumannii* | AAC(6')-I | 64 | 4 | 1 x 10⁸ | > 1 x 10⁸ |
| 3656 | *P. aeruginosa* | APH(3')-I + ANT(4')-II | 128 | 8 | 5 x 10⁷ | > 1 x 10⁸ |
| 3658 | *P. aeruginosa* | APH(3')-I + ANT(4')-II | >128 | 4 | > 1 x 10⁸ | > 1 x 10⁸ |
| 28233 | *P. aeruginosa* | APH(3')-I + AAC(3)-I | 4 | 1 | > 1 x 10⁸ | > 1 x 10⁸ |
| 3655 | *P. aeruginosa* | APH(3')-I + ANT(2") | 1 | 4 | > 1 x 10⁸ | > 1 x 10⁸ |

| | | | | | | |
|---|---|---|---|---|---|---|
| N-Acetyltransferases (AAC), O-Adenyltransferases (ANT), O-Phosphotransferases (APH) ^{a}EMB 30/30 refers to the EMBculture medium supplemented with amikacin and gentamicin, 30 µg/ml each. ^{b}MICs of amikacin and gentamicin were determined using the broth microdilution technique. ^{c}Underlined CFU counts are considered as results below cutoff values set at ≥ 1 x 10³ CFU/ml. | | | | | | |

### L.d.l.: Lowest detection limit.

Using an inoculum with an optical density of 0.5 McFarland (inoculum of ∼10⁸ CFU/ml), serial 10-fold dilutions of the isolates were made in normal saline and 100-µl portions were plated onto the SuperAminoglycoside medium (Table 2). To quantify the viable bacteria in each dilution, trypticase soy agar was inoculated concomitantly with 100 µl of suspension and incubated overnight at 37°C. The lowest limit of detection for the tested strains were determined using the SuperAminoglycoside medium (Table 2). The sensitivity and specificity cut-off values were set at 1 x 10³ CFU/ml i.e., a limit value of 1 x 10³ CFU/ml and above was considered as « not efficiently detected ».

### Results and Conclusions

All the 16S rRNA methylase producers grew on the SuperAminoglycoside medium in 24 h. Differentiation of several Enterobacteriaceael isolates could be made according to their color (Figs 2A and B). The lowest limit of detection was below the cut-off value for the 16S rRNA methylase producers while the limit of detection of the AG-susceptible strains and of the strains expressing aminoglycoside modifying enzymes was above, being ≥ 1 x 10⁶ CFU/ml (Table 3). The sensitivity and specificity of the SuperAminoglycoside medium for selecting amikacin- and gentamicin resistant isolates were 100% in both cases.

Similar results were obtained by using a Drigaliski-containing media (Figure 3) or with other AG combination or AG concentrations or switching from daptomycin to vancomycin. Spiked stools were also tested with a representative collection of fourteen isolates of various species expressing or not 16S rRNA methylases (Table 3) Spiked fecal samples were made by adding 100 µl of each strain dilution to 900 µl of fecal suspension that was obtained by suspending 5 g of freshly pooled feces from five healthy volunteers in 50 ml of distilled water. A non-spiked fecal suspension was used as negative control. The lowest detection limit was determined by plating 100 µl of each dilution on the screening medium. The sensitivity and specificity were determined using a cut-off value set at ≥ 10³ CFU/mL. This value may correspond to a low-level carriage of resistant bacteria in stools.

The spiked 16S rRNA methylase producers in stools grew with a lowest detection limit ranging from 10¹ to 10² CFU/ml (Table 3).

To assess the storage stability of the SuperAminoglycoside medium, *C*. *albicans, S. aureus* and AG-susceptible *E. coli* ATCC 25955 were subcultured daily onto the SuperAminoglycoside agar plates from a single batch of medium stored at 4°C. Growth of those isolates were consistently inhibited during at least a 7-days period.

The SuperAminoglycoside medium constitutes a screening medium aimed to detect multiple AG resistant bacteria and in particular the 16S rRNA methylase producers. This medium offers the possibility to select for those multidrug resistant *Enterobacteriaceae, Pseudomonas aeruginosa* and *A.baumannii.* It remains to be evaluated for the screening of other multiple AG resistant Gram negative species. It may be used in for detecting carriers of those multiple AG-resistant isolates

### References

1. Nordmann P, Naas T, Poirel L. 2011. Global spread of carbapenemase-producing Enterobacteriaceae. Emerg Infect Dis. 17:1791-8.
2. Falagas ME, Karageorgopoulos DE, Nordmann P. 2011. Therapeutic options for infections with Enterobacteriaceae producing carbapenem-hydrolyzing enzymes. Future Microbiol. 6:653-66.
3. Ramirez MS, Tolmasky ME. 2010. Aminoglycoside modifying enzymes. Drug Resist Updat. 13:151-71.
4. Doi Y, Wachino J, Arakawa Y. 2016. Aminoglycoside resistance: the emergence of acquired 16S ribosomal RNA methyltransferases. Infect Dis Clin North Am. 30:523-37.
5. Bercot B, Poirel L, Nordmann P. 2011. Updated multiplex polymerase chain reaction for detection of 16S rRNA methylases: high prevalence among NDM-1 producers. Diagn Microbiol Infect Dis. 71:442-5.
6. Wachino J, Shibayama K, Kurokawa H, Kimura K, Yamane K, Suzuki S, Shibata N, Ike Y, Arakawa Y. 2007. Novel plasmid-mediated 16S rRNA m1A1408 methyltransferase, NpmA, found in a clinically isolated Escherichia coli strain resistant to structurally diverse aminoglycosides. Antimicrob Agents Chemother. 51:4401-9.
7. Hugh R, Leifson E. 1953. The taxonomic significance of fermentative versus oxidative metabolism of carbohydrates by various gram negative bacteria. J Bacteriol. 66:24-
8. **Clinical Laboratory and Standard Institute (CLSI). 2012.** Methods for dilution of antimicrobial susceptibility tests for bacteria that grow aerobically. Approved standard 9th ed Document M07-A9 2012.
9. **Clinical Laboratory and Standard Institute (CLSI). 2014.** Performance standards for antimicrobial susceptibility testing. 24th informational supplement. CLSI document M100-S24. Clinical and Laboratory Standards Institute, Wayne, PA. 2014.
10. Rahman M, Prasad KN, Pathak A, Pati BK, Singh A, Ovejero CM, Ahmad S, Gonzalez-Zorn B. 2015. RmtC and RmtF 16S rRNA methyltransferase in NDM-1-producing Pseudomonas aeruginosa. Emerg Infect Dis. 21:2059-62.
11. Zhou Y, Yu H, Guo Q, Xu X, Ye X, Wu S, Guo Y, Wang M. 2010. Distribution of 16S rRNA methylases among different species of Gram-negative bacilli with high-level resistance to aminoglycosides. Eur J Clin Microbiol Infect Dis. 29:1349-53.
12. Haidar G, Alkroud A, Cheng S, Churilla TM, Churilla BM, Shields RK, Doi Y, Clancy CJ, Nguyen MH. 2016. Association between the presence of aminoglycoside-modifying enzymes and in vitro activity of gentamicin, tobramycin, amikacin, and plazomicin against Klebsiella pneumoniae carbapenemase- and extended-spectrum-beta-lactamase-producing Enterobacter species. Antimicrob Agents Chemother. 60:5208-14.
13. Zhanel GG, Lawson CD, Zelenitsky S, Findlay B, Schweizer F, Adam H, Walkty A, Rubinstein E, Gin AS, Hoban DJ, Lynch JP, Karlowsky JA. 2012. Comparison of the next-generation aminoglycoside plazomicin to gentamicin, tobramycin and amikacin. Expert Rev Anti Infect Ther. 10:459-73.

## Claims

1. A method for testing for Gram negative bacteria exhibiting resistance to multiple aminoglycoside antibiotics, wherein the method detects resistance conferred by the presence of 16S rRNA methylase while discriminating against aminoglycoside resistance due to other mechanisms, the method comprising:
- providing a medium and/or solution comprising at least two different aminoglycoside antibiotics and nutrients for supporting the growth of Gram negative bacteria;
- inoculating said medium and/or solution with a sample comprising bacteria;
- after incubation, screening for bacterial growth and/or metabolic activity in said medium and/or solution;
- finding a positive test outcome indicating said aminoglycoside resistance due to 16S rRNA methylase if bacterial growth and/or metabolic activity is detected.

2. The method of claim 1, wherein said method provides a negative test outcome if said bacteria exhibit aminoglycoside resistance exclusively due to aminoglycoside modifying activity, in particular selected from nucleotidyltransferase, phosphotransferase and acetyltransferase activity, and if said bacteria exhibit no aminoglycoside resistance.

3. The method of claim 1 or 2, wherein said at least two different aminoglycoside antibiotics comprise at least one 4,6-disubstituted deoxystreptamine aminoglycoside, preferably at least two 4,6-disubstituted deoxystreptamine aminoglycosides.

4. The method of any one of claims 1 to 3, wherein said at least two different aminoglycoside antibiotics comprise amikacin and gentamicin.

5. The method of any one of the preceding claims, wherein, in said medium and/or solution, each of said at least two different aminoglycoside antibiotics is independently present at a concentration of 0.1 to 100 mg/L, preferably 1 to 90 mg/L, more preferably 4 to 90 mg/L, and most preferably 10 to 80 mg/L.

6. The method of any one of claims 1 to 5, wherein said medium and/or solution comprises a color indicator, wherein a color of said indicator is pH dependent.

7. The method of any one of the preceding claims, wherein said nutrients comprise a carbohydrate source, and wherein metabolic activity of Gram negative, fermenting bacteria, in particular *Enterobacteriaceae,* exhibiting 16S rRNA methylase activity results in acidification of the solution due to metabolism of said carbohydrate source due to said metabolic activity.

8. The method of any one of the preceding claims, wherein said test outcome is determined in 3 hours or less starting from the inoculation of the medium and/or solution, and on the basis of a color of the medium and/or solution, wherein the test outcome is positive if a color indicator present in the medium and/or solution indicates an increase of the acidity of the medium and/or solution.

9. The method of any one of claims 1 to 7, wherein said medium is a solid medium, and wherein the test outcome is positive if growing colonies are detectable on the medium within 24 to 48 hours.

10. The method of claim 9, wherein said medium comprises, in addition to said at least two different aminoglycoside antibiotics, a daptomycin antibiotic and/or an antifungal agent.

11. The method of claim 9 and 10, which is for detecting fermenting and non-fermenting Gram negative bacteria, in particular, *Enterobacteriaceae, Pseudomonas aeruginosa* and *Acinetobacter baumannii*.

12. A test or medium for detecting Gram negative bacteria exhibiting resistance to aminoglycoside antibiotics, wherein the test or method detects resistance conferred by the presence of 16S rRNA methylase while discriminating against aminoglycoside resistance due to other mechanisms, said test comprising:
- at least two different aminoglycoside antibiotics; and
- nutrients for supporting the growth of Gram negative bacteria.

13. The test or medium of claim 12, which provides a negative test outcome if said bacteria exhibit aminoglycoside resistance exclusively due to aminoglycoside modifying activity, in particular selected from nucleotidyltransferase, phosphotransferase and acetyltransferase activity, and if said bacteria exhibit no aminoglycoside resistance.

14. The test or medium of any one of claims 12-13, wherein each of said at least two different aminoglycoside antibiotics is independently present at a concentration of 0.1 to 100 mg/L, preferably 1 to 90 mg/L, more preferably 4 to 90 mg/L, and most preferably 10 to 80 mg/L.

15. The test or medium of any one of claims 12-14, which further comprises at least one color indicator, wherein a color of said indicator is pH dependent, and wherein said nutrients comprise a carbohydrate source, and wherein metabolic activity of *Enterobacteriaceae* exhibiting 16S rRNA methylase activity results in acidification of the solution due to metabolism of said carbohydrate source due to said metabolic activity.

16. The test or medium of any one of claims 12-15, which comprises at least two solutions, a first solution comprising said at least two different aminoglycoside antibiotics and a second solution comprising said nutrients and a color indicator.

17. The test or medium of any one of claims 12-15, wherein said medium is a solid medium, and wherein the test outcome is positive if growing colonies are detectable on the medium within 24 to 48 hours.

18. The test or medium of any one of claims 12-15 and 17, wherein said medium comprises, in addition to said at least two different aminoglycoside antibiotics, a daptomycin antibiotic and/or an antifungal agent.

19. The test or medium of any one of claims 12-15 and 17-18, wherein said medium is for detecting Gram negative bacteria fermenting and non-fermenting bacteria, in particular, *Enterobacteriaceae, Pseudomonas aeruginosa* and *Acinetobacter baumannii*.
